# EUROPÄISCHE PATENTANMELDUNG

(11) **EP 2 014 168 A1**
(43) Veröffentlichungstag der Anmeldung: **14.01.2009**
(21) Anmeldenummer: 07011962.3
(22) Anmeldetag: 19.06.2007
(51) Int. Cl.: A01N 43/76, A01N 43/90, C07D 413/12

(54) **Insektizide heterocyclische Verbindungen**

(71) Anmelder: Bayer CropScience AG, 40789 Monheim (DE)
(72) Erfinder: Die Erfindernennung liegt noch nicht vor

(57) **Zusammenfassung**

Die vorliegende Anmeldung betrifft neue insektizide heterocyclische Verbindungen, Verfahren zu Ihrer Herstellung und ihre Verwendung zur Bekämpfung von tierischen Schädlingen, vor allem von Arthropoden, insbesondere Insekten,

## Beschreibung

Die vorliegende Anmeldung betrifft neue insektizide heterocyclische Verbindungen, Verfahren zu ihrer Herstellung und ihre Verwendung zur Bekämpfung von tierischen Schädlingen, vor allem von Arthropoden, insbesondere Insekten.

Aus der WO 2007/202377 sind bereits bestimmte heterocyclische Verbindungen mit insektizider Wirkung bekannt.

Bekannt sind weiterhin aus der DE 2443188 folgende Verbindungen 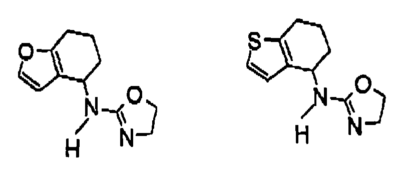 mit pharmakologischen Eigenschaften,

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet worden und es besteht ständig Bedarf an neuen Verbindungen mit gegenüber den bekannten Verbindungen zumindest in Bezug auf einzelne Aspekte verbesserten Eigenschaften

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue

Verbindungen der Formel (I) 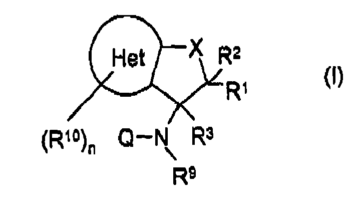 in welcher
- Het: für einen 5- oder 6-gliedrigen heterocyclischen Ring steht,
- X: für -O-, -SOₘ-, -NR⁴-, -CR⁵R⁶-, -CR⁵R⁶-CR⁷R⁸-, -O-CR⁷R⁸-, -SOₘ-CR⁷R⁸-, -NR⁴-CR⁷R⁸-, - CR⁵R⁶-O-, -CR⁵R⁶-SOₘ-, -CR⁵R⁶-NR⁴-, -SOₘNR⁴-, -NR⁴-SOₘ-, -NR⁴-O- oder -O-NR⁴-steht,
- Q: für eine der Gruppen A, B, C oder D steht 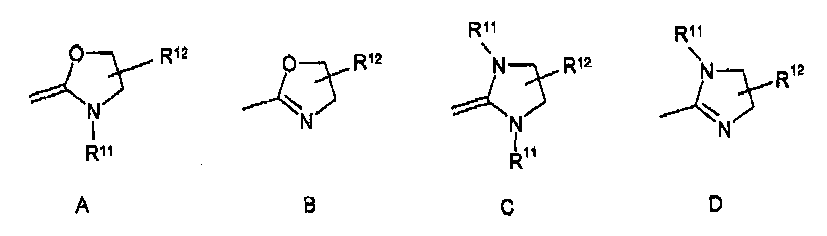
wobei
- R⁹: in den Gruppen A und C mit dem verknüpfenden Kohlenstoffatom eine Doppelbindung bildet,
oder
- R⁹: für Wasserstoff, Cyano, Nitro, Hydroxy, Alkoxy, Amino, Alkylamino, Arylalkyl, Hetarylalkyl, Hetaryl, tri(C₁-C₆-Alkyl)silyl, -C(Y)R¹³, -C(Y)OR¹⁴, -C(Y)SR¹⁴, -SOₘR¹⁴, - C(Y)NR¹⁵R¹⁶, -SOₘNR¹⁵R¹⁶, -P(Y)(YR¹⁷)(OR¹⁸), P(Y)(NR¹⁹R²⁰)(NR²¹R²²), -CHR²³-Y-R²⁴, -CH=NR²⁵ oder -C(=NR²⁶)(SR²⁷) steht,
- R¹, R², R⁴, R⁵, R⁶, R⁷ und R⁸: unabhängig von einander für Wasserstoff, Cyano, Nitro, Hydroxy oder für jeweils gegebenenfalls durch Halogen, Alkoxy oder Alkylthio substituiertes Alkyl, Alkenyl, Alkinyl, Alkoxy oder Alkylthio stehen,
- R³: für Wasserstoff, Cyano, Carboxy, Carboxyalkyl, Carbamoyl, Carbamoylalkyl oder für jeweils gegebenenfalls durch Halogen, Alkoxy oder Alkylthio substituiertes Alkyl, Alkenyl oder Alkinyl steht,
- R¹¹: für Wasserstoff, Cyano, Nitro, Hydroxy, Alkoxy, Amino, Alkylamino, Arylalkyl, Hetarylalkyl, Hetaryl, tri(C₁-C₆-Alkyl)silyl, -C(Y)R¹³, -C(Y)OR¹⁴, -C(Y)SR¹⁴, -SOₘR¹⁴, - C(Y)NR¹⁵R¹⁶, -SOₘNR¹⁵R¹⁶, -P(Y)(YR¹⁷)(OR¹⁸), P(Y)(NR¹⁹R²⁰)(NR²¹R²²), -CHR²³-Y-R²⁴, -CH=NR²⁵ oder -C(=NR²⁶)(SR²⁷) steht,
- Y: für Sauerstoff oder Schwefel steht,
- R¹⁰: für Wasserstoff, Halogen, Cyano, Sauerstoff (beispielsweise eine Ketogruppe, ein N-Oxid, ein Sulfoxid oder Sulfon bildend), Hydroxy, Alkyl, Alkoxy, Haloalkyl, Alkoxyalkyl, Alkoxyalkoxyalkyl, Cycloalkyl, Cyanoalkyl, Haloalkoxy, Alkylthio, Haloalkylthio, Alkylsulfonyl, Alkylsulfonyloxy, Halogenalkylsulfonyl, Halogenalkylsufonyloxy, Alkoxycarbonyl, Acetyl, Alkylcarbonyl, Alkenylcarbonyl, Pentafluorosulfanyl, Amino, Mono- und Dialkylamino, Cycloalkylamino, Alkenyl, Haloalkenyl, Alkinyl, Haloalkinyl, Cyano oder Nitro oder für jeweils gegebenenfalls durch Halogen, Alkyl, Haloalkyl, Alkoxy, Haloalkoxy, Alkoxycarbonyl, Nitro oder Cyano substituiertes Aryl, Aryloxy oder Hetaryl steht,
- n: für eine ganze Zahl aus der Reihe 1, 2, 3 und 4 steht,
- R¹²: für Wasserstoff, Cyano, Halogen oder für gegebenenfalls durch Halogen, Alkoxy oder Alkylthio substituiertes Alkyl steht,
- R¹³: und R¹⁴ unabhängig voneinander für Wasserstoff, Alkyl, Haloalkyl, Alkoxyalkyl, Alkoxyalkylcarbonyl, Alkenyl oder Alkinyl stehen,
- R¹⁵ und R¹⁶: unabhängig voneinander für Wasserstoff oder für jeweils gegebenenfalls durch Halogen, Alkyl, Alkoxy, Cyano, Nitro, S(O)ₘ-alkyl, S(O)ₘ-Halogenalkyl substituiertes Alkyl, Cycloalkylalkyl, Alkylcycloalkyl, Alkenyl, Alkinyl, Arylalkyl, Heterocyclylalkyl, Hetarylalkyl, Aryl, Heterocyclyl, Hetaryl, Alkylcarbonyl, Alkoxycarbonyl, Alkoxycarbonylalkyl, Alkylthioalkyl, Alkylsulfinylalkyl, Alkylsulfonylalkyl, Mono- oder Dialkylaminocarbonylalkyl, Aminoalkyl, Mono oder Dialkylaminoalkyl oder Arylcarbonyl stehen,
- R¹⁷ und R¹⁸: unabhängig voneinander für Wasserstoff, Alkyl, Haloalkyl, Alkenyl oder Alkinyl stehen,
- R¹⁹, R²⁰, R²¹ und R²²: unabhängig voneinander Wasserstoff, Alkyl oder Alkenyl stehen,
- R²³: für Wasserstoff oder Alkyl steht,
- R²⁴: für Alkyl, Haloalkyl, C(Y)R²⁸ oder P(Y)(OR²⁹)(OR³⁰) steht,
- R²⁵: für Wasserstoff, Alkyl, Haloalkyl, Hydroxy, Alkoxy, Haloalkoxy, Cyano, Hetarylalkyl oder Hetaryl steht,
- R²⁶ und R²⁷: unabhängig voneinander für Wasserstoff, Alkyl, Aralkyl, -C(Y)R¹³, -C(Y)OR¹⁴, - C(Y)SR¹⁴, -SOₘR¹⁴, -C(Y)NR¹⁵R¹⁶, -SOₘNR¹⁵R¹⁶, -P(Y)(YR¹⁷)(OR¹⁸), P(Y)(NR¹⁹R²⁰)(NR²¹R²²) oder-CHR²³-Y-R²⁴ stehen,
- R²⁸: für Wasserstoff, Alkyl oder Haloalkyl steht,
- R²⁹ und R³⁰: unabhängig voneinander für Wasserstoff, Alkyl oder Haloalkyl stehen und
- m: für eine ganze Zahl aus der Reihe 0,1 und 2 steht,
mit der Maßgabe, dass Het nicht für Furan oder Thiophen steht, wenn X für CR⁵R⁶CR⁷R⁸ und Q für die Gruppe B steht und R¹, R², R³, R⁵, R⁶, R⁷, R⁸, R⁹, R¹⁰ und R¹² gleichzeitig für Wasserstoff stehen.

Weiter wurde gefunden, dass sich Verbindungen der Formel (I) herstellen lassen, indem man (Verfahren 1) Verbindungen der Formel (II) 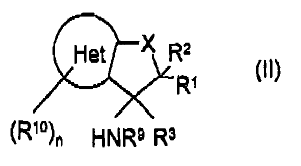 in welcher
Het, X, R¹, R², R³, R⁹, R¹⁰ und n die oben angegebenen Bedeutungen haben mit einem 2-Chlorethylisocyanat der Formel (III) 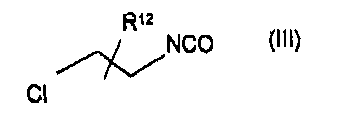 in welcher
R¹² die oben angegebene Bedeutung hat, in Gegenwart eines Verdünnungsmittels umsetzt und das erhaltene Reaktionsprodukt der Formel (IV) 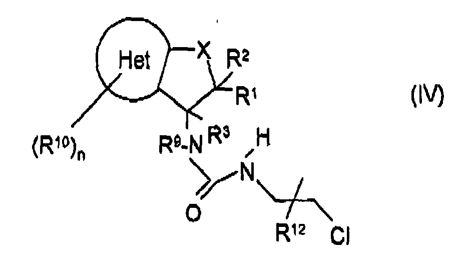 in Gegenwart einen Verdünnungsmittels und einer Base zu einer Verbindung der Formel (I B) umsetzt 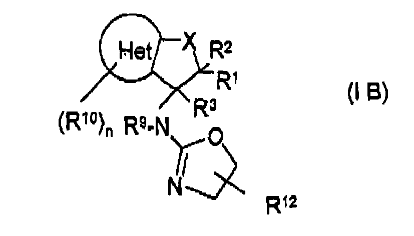 in welcher
Het, X, R¹, R², R³, R⁹, R¹⁰, R¹² und n die oben angegebenen Bedeutungen haben,
und diese gegebenenfalls anschließend, für den Fall R⁹ = H, mit für die Einführung des Restes R¹¹ geeigneten Verbindungen zu einer Verbindung der Formel (I A) 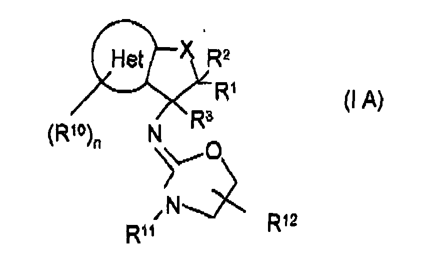 in welcher
Het, X, R¹, R², R³, R¹⁰, R¹¹, R¹² und n die oben angegebenen Bedeutungen haben,
umsetzt.

Verbindungen der Formel (I) erhält man auch (Verfahren 2), indem man die oben genannten Verbindungen der Formel (II) mit Verbindungen der Formel (V) 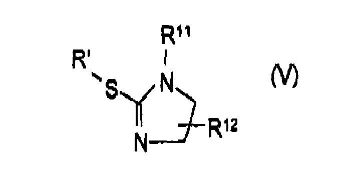 worin
R¹¹ und R¹² die oben angegebenen Bedeutungen haben und R' für Alkyl steht,
zu Verbindungen der Formel (I D) 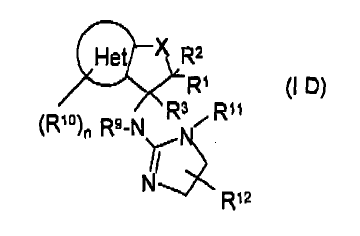 in welcher
Het, X, R¹, R², R³, R⁹, R¹⁰, R¹¹, R¹² und n die oben angegebenen Bedeutungen haben, umsetzt und die Verbindungen der Formel (I D) anschließend, für den Fall R⁹ = H, mit für die Einführung des Restes R¹¹ geeigneten Verbindungen zu den Verbindungen der Formel (I C) 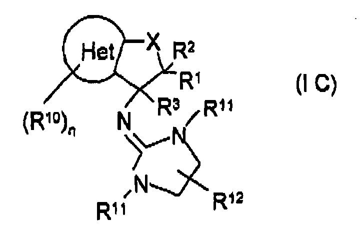 in welcher
Het, X, R¹, R², R³, R¹⁰, R¹¹ und n die oben angegebenen Bedeutungen haben,
umsetzt.

Schließlich wurde gefunden, das die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische oder in unterschiedlichen tautomeren Formen in unterschiedlicher Zusammensetzung vorliegen. Die Erfindung betrifft sowohl die reinen Isomere als auch die Isomerengemische,

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im Folgenden erläutert.
- Het: steht bevorzugt für einen 5- oder 6-gliedrigen heterocyclischen Ring, in welchem ein bis vier Ringglieder unabhängig von einander Sauerstoff, Schwefel, Stickstoff oder durch den Rest R¹⁰ substituierter Stickstoff sein können, beispielsweise für Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, I-sothiazolyl, 1,2,3-Oxadiazolyl, 1,2,4-Oxadiazolyl, 1,3,4-Oxadiamlyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
- X: steht bevorzugt für -O-, -SOₘ-, -NR⁴-, -CR⁵R⁶-, -CR⁵R⁶-CR⁷R⁸-, -O-CR⁷R⁸-, -SOₘ-CR⁷R⁸-, -NR⁴-CR⁷R⁸-, -CR⁵R⁶-O-, -CR⁵R⁶-SOₘ-, -CR⁵R⁶-NR⁴-, -SOₘNR⁴-, -NR⁴-SOₘ-, -NR⁴-O-oder -O-NR⁴-.
- Q: steht bevorzugt für eine der Gruppen A bis D 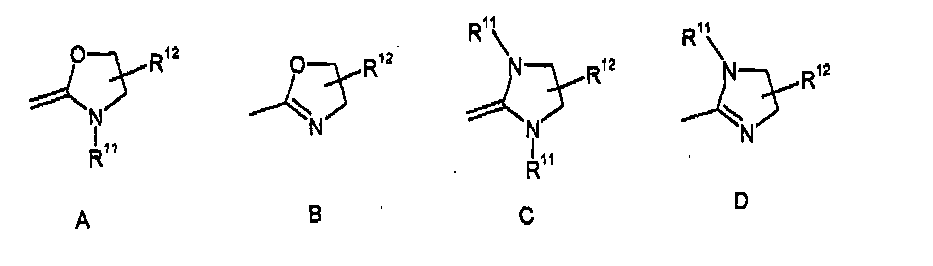
wobei
- R⁹: in den Gruppen A und C mit dem verknüpfenden Kohlenstoffatom eine Doppelbindung bildet,
oder
- R⁹: steht bevorzugt für Wasserstoff, Cyano, -C(Y)R¹³, C(Y)OR¹⁴, -C(Y)SR¹⁴, -C(Y)NR¹⁵R¹⁶, - P(Y)(YR¹⁷)(OR¹⁸) oder -P(Y)(NR¹⁹R²⁰)(NR²¹R²²).
- R¹, R², R⁴, R⁵, R⁶, R⁷ und R⁸: stehen unabhängig voneinander bevorzugt für Wasserstoff, Cyano, Nitro, Hydroxy oder für gegebenenfalls durch Halogen, C₁-C₆-Alkoxy oder C₁-C₆-Alkylthio substituiertes C₁-C₆-Alkyl, C₂-C₆-Alkenyl, C₂-C₆-Alkinyl, C₁-C₆-Alkoxy oder C₁-C₆-Alkylthio.
- R³: steht bevorzugt Wasserstoff, Cyano, Carboxy, Carboxyalkyl, Carbamoyl, Carbamoyl-C₁-C₆-alkyl oder für jeweils gegebenenfalls durch Halogen, C₁-C₆-Alkoxy oder C₁-C₆-Alkylthio substituiertes C₁-C₆-Alkyl, C₂-C₆-Alkenyl oder C₂-C₆-Alkinyl.
- R¹¹: steht bevorzugt für Wasserstoff, Cyano, -C(Y)R¹³, -C(Y)OR¹⁴, -C(Y)SR¹⁴, -C(Y)NR¹⁵R¹⁶, - P(Y)(YR¹⁷)(OR¹⁸), oder -P(Y)(NR¹⁹R²⁰)(NR²¹R²²).
- Y: steht bevorzugt für Sauerstoff oder Schwefel.
- R¹⁰: steht bevorzugt für Wasserstoff, Halogen, Cyano, Sauerstoff, C₁-C₆-Alkyl, C₁-C₆-Alkoxy, C₁-C₆-Haloalkyl, C₁-C₆-Haloalkoxy, C₁-C₆-Alkylthio, C₁-C₆-Haloalkylthio, C₁-C₆-Alkylsulfonyl, C₁-C₆-Alkylsulfonyloxy, C₁-C₆-Halogenalkylsulfonyl oder C₁-C₆-Halogenalkylsufonyloxy.
- n: steht bevorzugt für eine ganze Zahl aus der Reihe 1, 2, 3 und 4.
- R¹²: steht bevorzugt für Wasserstoff oder C₁-C₆-Alkyl.
- R¹³ und R¹⁴: stehen unabhängig voneinander bevorzugt für Wasserstoff, C₁-C₆-Alkyl, C₁-C₆-Haloalkyl, C₂-C₆-Alkenyl oder C₂-C₆-Alkinyl.
- R¹⁵ und R¹⁶: stehen unabhängig voneinander bevorzugt für Wasserstoff oder für jeweils gegebenenfalls durch Halogen, C₁-C₆-Alkyl, C₁-C₆-Alkoxy, Cyano, Nitro, S(O)ₘ- C₁-C₆-Alkyl oder S(O)ₘ-C₁-C₆-Halogenalkyl substituiertes C₁-C₆-Alkyl, C₃-C₇-Cycloalkyl-C₁-C₆-alkyl, C₁-C₆-Alkyl-C₃-C₇-cycloalkyl, C₂-C₆-Alkenyl, C₂-C₆-Alkinyl, Aryl-C₁-C₆-alkyl, Heterocyclyl-C₁-C₆-alkyl, Aryl, Heterocyclyl, C₁-C₆-Alkylcarbonyl, C₁-C₆-Alkoxymbonyl, C₁-C₆-Alkoxycarbonyl-C₁-C₆-alkyl, C₁-C₆-Alkylthio-C₁-C₆-alkyl, C₁-C₆-Alkylsulfinyl-C₁-C₆-alkyl, C₁-C₆-Alkyl-sulfonyl-C₁-C₆-alkyl, Mono- oder Di-C₁-C₆-alkylaminocarbonyl-C₁-C₆-alkyl, Amino-C₁-C₆-alkyl, Mono oder Di-C₁-C₆-alkylamino-C₁-C₆-alkyl, wobei Aryl insbesondere für Phenyl und Heterocyclyl insbesondere für 5- und 6-gliedrige, Sauerstoff, Stickstoff oder Schwefel enthaltende Heterocyclen steht.
- R¹⁷ und R¹⁸: stehen unabhängig voneinander bevorzugt für Wasserstoff, C₁-C₆-Alkyl, C₁-C₆-Haloalkyl oder C₂-C₆-Alkonyl,
- R¹⁹, R²⁰, R²¹ und R²²: stehen unabhängig voneinander bevorzugt für Wasserstoff oder C₁-C₆-Alkyl,
- m: steht bevorzugt für eine ganze Zahl aus der Reihe 0,1 und 2.
- Het: steht besonders bevorzugt für einen 5- oder 6-gliedrigen heterocyclischen Ring, in welchem ein bis drei Ringglieder unabhängig von einander O, S, N oder N-R¹⁰ sein können, jedoch nicht mehr als ein Sauerstoff- oder Schwefelatom im Ring vorhanden sind, beispielsweise für Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiamlyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
- Het: steht ganz besonders bevorzugt für Furyl, Thienyl, Pyrrolyl, Pyrazolyl, Pyridyl, Pyrimidinyl, Oxazolyl, Isoxazolyl und Thiazolyl,
- X: steht besonders bevorzugt für -O-, -SOₘ-, -NR⁴-, -CR⁵R⁶-, -CR⁵R⁶-CR⁷R⁸-, -O-CR⁷R⁸-, - SOₘ-CR⁷R⁸-, -NR⁴-CR⁷R⁸-, -CR⁵R⁶-O-, -CR⁵R⁶-SOₘ-, -CR⁵R⁶-NR⁴-, -SOₘNR⁴-, -NR⁴-SOₘ-, -NR⁴-O- oder -O-NR⁴-.
- Q: steht besonders bevorzugt für eine der Gruppen A oder B 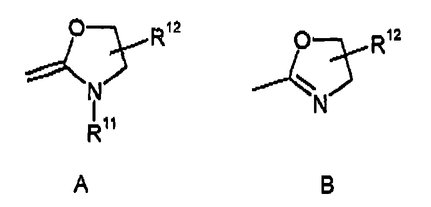
wobei
- R⁹: in der Gruppe A mit dem verknüpfenden Kohlenstoffatom eine Doppelbindung bildet oder
- R⁹: steht ganz besonders bevorzugt für Wasserstoff, Cyano, -C(Y)R¹³, -C(Y)OR¹⁴, -C(Y)SR¹⁴, -C(Y)NR¹⁵R¹⁶, -P(Y)(YR¹⁷)(OR¹⁸) oder -P(Y)(NR¹⁹R²⁰)(NR²¹R²²).
- R¹, R², R⁴, R⁵, R⁶, R⁷ und R⁸: stehen unabhängig voneinander besonders bevorzugt für Wasserstoff, Cyano, Nitro, Hydroxy oder für gegebenenfalls durch Halogen, C₁-C₄-Alkoxy oder C₁-C₄-Alkylthio substituiertes C₁-C₄-Alkyl oder C₂-C₄-Alkenyl.
- R³: steht besonders bevorzugt für Wasserstoff, Cyano, Carboxy, Carboxyalkyl, Carbamoyl, C₁-C₄-Carbamoylalkyl oder für gegebenenfalls durch Halogen, C₁-C₄-Alkoxy oder C₁-C₄-Alkylthio substituiertes C₁-C₄-Alkyl, C₂-C₄-Alkenyl oder C₂-C₄-Alkinyl,
- R¹¹: steht besonders bevorzugt für Wasserstoff, Cyano, -C(Y)R¹³, -C(Y)OR¹⁴, -C(Y)SR¹⁴, -C(Y)NR¹⁵R¹⁶, -P(Y)(OR¹⁷)(OR¹⁸) oder -P(Y)(NR¹⁹R²⁰)(NR²¹R²²),
- Y: steht besonders bevorzugt für Sauerstoff oder Schwefel.
- R¹⁰: steht besonders bevorzugt für Wasserstoff, Halogen, Cyano, Sauerstoff, C₁-C₄-Alkyl, C₁-C₄-Alkoxy, C₁-C₄-Haloalkyl, C₁-C₄-Haloalkoxy, C₁-C₄-Alkylthio, C₁-C₄-Haloalkylthio, C₁-C₄-Alkylsulfonyl, C₁-C₄-Alkylsulfonyloxy, C₁-C₄-Halogenalkylsulfonyl oder C₁-C₄-Halogenalkylsufonyloxy,
- n: steht besonders bevorzugt für eine ganze Zahl aus der Reihe 1, 2, 3 und 4.
- R¹²: steht besonders bevorzugt für Wasserstoff oder C₁-C₄-Alkyl.
- R¹³ und: R¹⁴ stehen unabhängig voneinander besonders bevorzugt für Wasserstoff, C₁-C₄-Alkyl, C₁-C₄-Haloalkyl, C₂-C₄-Alkenyl oder C₂-C₄-Alkinyl.
- R¹⁵ und R¹⁶: stehen unabhängig voneinander besonders bevorzugt für Wasserstoff oder für jeweils gegebenenfalls durch Halogen, C₁-C₄-Alkyl, C₁-C₄-Alkoxy, Cyano, S(O)ₘC₁-C₄-Alkyl oder S(O)ₘ-C₁-C₄-Halogenalkyl substituiertes C₁-C₄-Alkyl, C₃-C₆-Cycloalkyl-C₁-C₄-alkyl, C₁-C₄-Alkyl-C₃-C₇-cycloalkyl, C₂-C₄-Alkenyl, C₂-C₄-Alkinyl, Aryl-C₁-C₄-alkyl, Heterocyclyl-C₁-C₄-alkyl, Aryl, Heterocyclyl, C₁-C₄-Alkylcarbonyl, C₁-C₄-Alkoxycarbonyl, C₁-C₄-Alkoxycarbonyl-C₁-C₄-alkyl, C₁-C₄-Alkylthio-C₁-C₄-alkyl, C₁-C₄-Alkylsulfinyl-C₁-C₄-alkyl, C₁-C₄-Alkylsulfonyl-C₁C₄-alkyl, Mono- oder Di-C₁-C₄-alkylaminocarbonyl-C₁-C₄-alkyl, C₁-C₄-Aminoalkyl, Mono- oder Di-C₁-C₄-alkylamino-C₁-C₄-alkyl, wobei Aryl insbesondere für Phenyl und Heterocyclyl insbesondere für 5- und 6-gliedrige, Sauerstoff, Stickstoff oder Schwefel enthaltende Heterocyclen steht.
- R¹⁷ und R¹⁸: stehen unabhängig voneinander besonders bevorzugt für Wasserstoff, C₁-C₄-,Alkyl, C₁-C₄-Haloalkyl oder C₂-C₄-Alkenyl,
- R¹⁹, R²⁰, R²¹ und R²²: stehen unabhängig voneinander besonders bevorzugt für Wasserstoff oder C₁-C₄-Alkyl.
- m: steht besonders bevorzugt für eine Zahl aus der Reihe 0, 1, und 2.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedofinitionen können untereinander also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Setzt man bei dem erfindungsgemäßen Verfahren 1 zur Herstellung der neuen Verbindungen der Formel (I) als Verbindung der Formel (II) beispielsweise 5,6-Dihydro-4H-cyclopenta[b]thiophen-6-amine Hydrochlorid und als Verbindung der Formel (III) Chlorethylisocyanat ein, so lässt sich die erste Stufe des Herstellungsverfahrens 1 durch folgendes Reaktionsschema I wiedergeben:

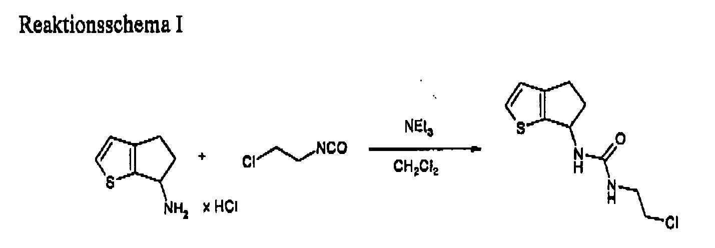

Die erste Stufe des Herstellungsverfahrens 1 wird im Allgemeinen so durchgeführt, dass man Verbindungen der Formel (II) mit Verbindungen der Formel (III) in Gegenwart eines basischen Hilfsmittels in einem Verdünnungsmittel umsetzt.

Als basische Hilfsmittel kommen Amine, beispielsweise Trialkylamine in Frage. Genannt sei Triethylamin.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Verbindungen der Formel (II) können z. T. kommerziell oder nach literaturbekannten Methoden erhalten werden (vgl. z.B. Heterocycles 26, 3233 (1987), Ars Pharmaceutica 27, 167 (1986)).

Die weiterhin für die Durchführung des erfindungsgemäßen Verfahrens 1 als Ausgangsstoffe zu verwendenden Verbindungen sind allgemein durch die Formel (III) definiert.

In der Formel (III) hat R¹² die Bedeutungen, die bereits im Zusammenhang mit der Beschreibung der erfindungsgemäßen Stoffe der Formel (I) genannt wurden.

Die Verbindungen der Formel (III) können z. T. kommerziell oder nach literaturbekannten Methoden erhalten werden

Setzt man in der zweiten Stufe des erfindungsgemäßen Verfahrens 1 zur Herstellung der neuen Verbindungen der Formel (I) als Verbindung der Formel (IV) beispielsweise den nach Reaktionsschema I erhaltenen 1-(2-Chloroethyl)-3-(5,6-dihydro-4H-cyclopenta[b]thien-6-yl)-harnstoff ein, so lässt sich die zweite Stufe des Herstellungsverfahrens 1 durch folgendes Reaktionsschema II wiedergeben:

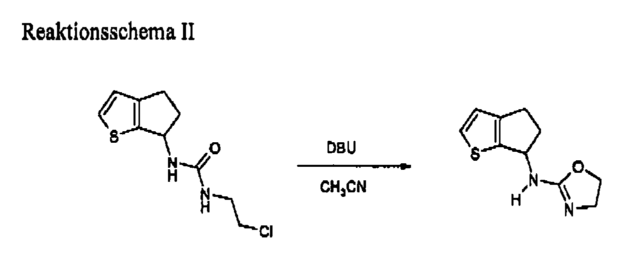

In der zweiten Stufe des Verfahrens 1 wird die nach Stufe 1 erhaltene Verbindung (IV) in einem geeignetenVerdünnungsmittel wie z. B. Akoholen, Ethern oder Wasser, oder einer Mischung der jeweiligen Verdünnungsmittel gegebenenfalls in Gegenwart einer Base bei einer Temperatur von -20 °C bis +100 °C zur Reaktion gebracht.

Geeignete Basen sind beispielsweise anorganische oder organische, Stickstoff enthaltende Basen, insbesondere Trialkylamine, wie beispielsweise Triethylamin , oder cyclische tertiäre Amine, wie beispielsweise Diazabicycloundecan oder Diazabicyclooctan,

Setzt man in der dritten Stufe des erfindungsgemäßen Verfahrens 1 zur Herstellung der neuen Verbindungen der Formel (I) als Verbindung der Formel (IB) beispielsweise das nach Reaktionsschema II erhaltene N-(5,6-Dihydro-4H-cyclopenta[b]thien-6-yl)-4,5-dihydro-1,3-oxazol-2-amin und als für die Einführung des Restes R¹¹ geeignete Verbindung 2-Methoxyethylisothiocyanat ein, so lässt sich die dritte Stufe des Herstellungsverfahrens 1 durch folgendes Reaktionsschema III wiedergeben:

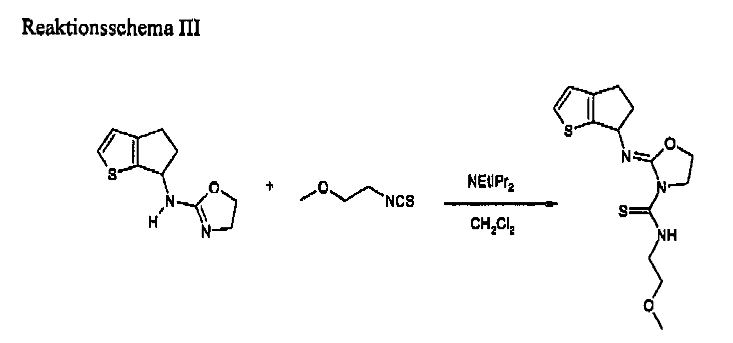

Für die Einführung des Restes R¹¹ geeignete Verbindungen sind beispielsweise Isothiocyanate, die in einem Verdünnungsmittel gegebenenfalls in Gegenwart einer Base mit einer Verbindung der Formel (I B) umgesetzt werden.

Geeignete Basen sind beispielsweise organische, Stickstoff enthaltende Basen, insbesondere Trialkylamine, wie beispielsweise Triethylamin, oder cyclische tertiäre Amine, wie beispielsweise Diazabicycloundecan oder Diazabicyclooctan.

Andere für die Einführung des Restes R¹¹ geeignete Verbindungen sind beispielsweise primäre und sekundäre Amine.

Hierbei setzt man in der dritten Stufe des erfindungsgemäßen Verfahrens 1 zur Herstellung der neuen Verbindungen der Formel (I) als Verbindung der Formel (IB) beispielsweise das nach Reaktionsschema II erhaltene N-(5,6-Dihydro-4H-cyclopenta[b]thien-6-yl)-4,5-dihydro-1,3-oxazol-2-amin und als für die Einführung des Restes R¹¹ geeignete Verbindung 2-Aminoethanol und 1,1'-Carbonothioylbis(1H-imidazole) ein, so lässt sich die dritte Stufe des Herstellungsverfahrens 1 durch folgendes Reaktionsschema IV wiedergeben:

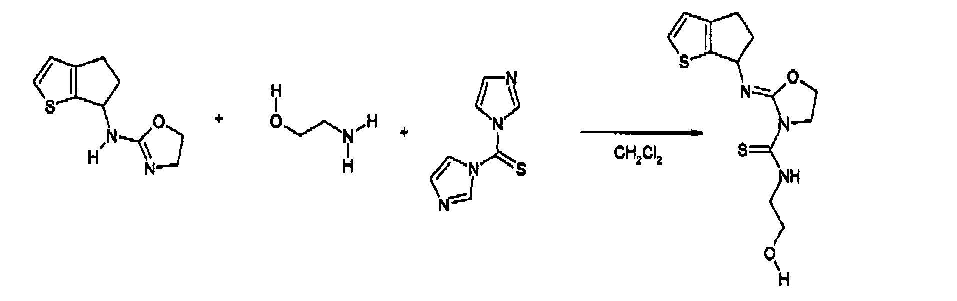

Im Allgemeinen ist es vorteilhaft, die jeweilige Stufe des erfindungsgemäßen Herstellungsverfahrens 1 in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 1 kommen unter den Reaktionsbedingungen inerte organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, n-Butylether, Anisol, Phenetol, Cyclohexylmethylether, Dimethylether, Diethylether, Dipropylether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methylmorpholin, Pyridin und Tetramethylendiamin; Nitrokohlenwasserstoffe wie Nitromethan, Nitroethan, Nitropropan, Nitrobenzol, Chlornitrobenzol, o-Nitrotoluol, Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N*-Methyl-formamid, N,N-Dimethyl-formamid, *N,N*-Dipropyl-formamid, *N,N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin; Ketone wie Aceton, Acetophenon, Methylethylketon, Methylbutylketon.

Man kann in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind chlorierte Kohlenwasserstoffe wie beispielsweise Methylenchlorid oder Nitrile wie z.B Acetonitril.

Die erste Stufe des erfindungsgemäßen Verfahrens 2 kann analog zu dem in EP 0 251 453 beschriebenen Verfahren durchgeführt werden, während die zweite Stufe analog zur dritten Stufe des Verfahrens 1 ausgeführt werden kann.

Die für die erste Stufe des Verfahrens 2 benötigten Verbindungen der Formel (V) sind kommerziell erhältlich oder können nach bekannten Methoden hergestellt werden (vgl. J. Amer. Chem. Soc. 73, 602, 1951).

Für die Herstellung der Verbindungen der Formel (I) wird auch auf die Herstellungsbeispiele verwiesen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp.
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp.
Aus der Ordnung der Chilopoda z.B. Geophilus spp., Scutigera spp.
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa docemlineata, Lissorhoptrus oryzophilus, Lixus spp., Lyctus spp., Meligethes aeneus, Melolontha mololontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp., Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp.
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
Weiterhin lassen sich Protozoen, wie Eimeria, bekämpfen.
Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.
Aus der Ordnung der Homoptera z.B. Acyrthosipon spp., Aeneolamia spp., Agonoscena spp., A-leurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii,
Aus der Ordnung der Hymenoptera z.B. Diprion spp., Hoplocampa spp., Lasius spp., Monomorium pharaonis, Vespa spp.
Aus der Ordnung der Isopoda z.B. Armadillidium vulgare, Oniscus asellus, Porcellio scaber.
Aus der Ordnung der Isoptera z.B. Reticulitermes spp., Odontotermes spp.
Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.
Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
Aus der Ordnung der Siphonaptera z.B. Ceratophyllus spp., Xenopsylla cheopis.
Aus der Ordnung der Symphyla z.B. Scutigerella immaculata.
Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.
Aus der Ordnung der Thysanura z.B. Lepisma saccharina.
Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.
Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.
Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.
Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung,
Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.
Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbrnzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).
Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew:-% liegen,

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs), Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die hoterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw, Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryI-IIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B.

Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B, Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto-und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.
Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.
Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.
Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.
Außerdem wurde gefunden, daß die erfindungsgemäßen Verbindungen eine hohe insektizide Wirkung gegen Insekten zeigen, die technische Materialien zerstören.
Beispielhaft und vorzugsweise - ohne jedoch zu limitieren - seien die folgenden Insekten genannt:
   Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
   Hautflügler wie Sirexjuvencus, Urocerus gigas, Urocerus gigas taignus, Urocerus augur;
   Termiten wie Kalotermes flavicollis, Cryptotermes brevis, Heterotermes indicola, Reticulitermes flavipes, Reticulitermes santonensis, Rotirulitermes lucifugus, Mastotermes darwiniensis, Zootermopsis nevadensis, Coptotermes formosanus;
   Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsoktizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus,

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionelia, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel 1 (Vorstufe)

### 1-(2-Chloroethyl)-3-(5,6-dihydro-4H-cyclopenta[b]thien-6-yl)-harnstoff

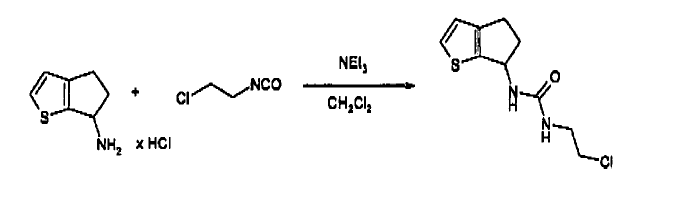

Eine Lösung von 2 g (11,4 mmol) 5,6-Dihydro-4H-cyclopenta[b]thiophen-6-amine Hydrochlorid und 2.5g (25 mmol) Triethylamin in 40 ml Dichlorethan wird auf 0°C abgekühlt und anschließend 1.33g (12.6 mmol) Chlorethylisocyanat gelöst in 2 ml Dichlormethan zugetropft. Nach drei Stunden werden die flüchtigen Bestandteile abdestilliert, der verbleibende Rückstand mit Hexan verrührt und so der 1-(2-chloroethyl)-3-(5,6-dihydro-4H-cyclopenta[b]thien-6-yl)-harnstoff als kristalliner Feststoff erhalten.
3g (94 % d.Theorie), logP (Ameisensäure) 1,77.

### Beispiel 2

### N-(5,6-Dihydro-4H-cyclopenta[b]thien-6-yl)-4,5-dihydro-1,3-oxazol-2-amin

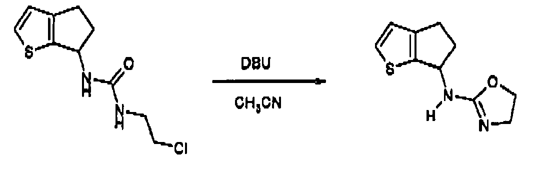

3 g (12.3 mmol) 1-(2-Chloroethyl)-3-(5,6-dihydro-4H-cyclopenta[b]thien-6-yl)-harnstoff und 2.3 g (15.3 mmol) 1,8-Diazabicyclo(5,4,0)undec-7-en in 50 ml Acetonotril werden 18 Stunden bei 45°C gerührt. Anschließend werden die flüchtigen Bestandteile abdestilliert, der verbleibende Rückstand in Essigsäureethylester gelöst, dreimal mit Wasser gewaschen und über Natriumsulfat getrocknet. Nach dem Abfiltrieren wird das Lösungsmittel abgezogen und der Rückstand mit Diethylether/Petrolother verrieben. Dabei fallen 1.4 g (55 % d.Theorie) N-(5,6-Dihydro-4H-cyclopenta[b]thien-6-yl)-4,5-dihydro-1,3-oxazol-2-amine an,
logP (Ameisensäure) 0.42 ¹H-NMR (DMSO-d6): 4.97 t

### Beispiel 3

### (2Z)-2-(5,6-Dihydro-4H-cyclopenta[b]thien-6-ylimino)-N-(2-methoxyethyl)-1,3-oxazolidine-3-carbothioamid

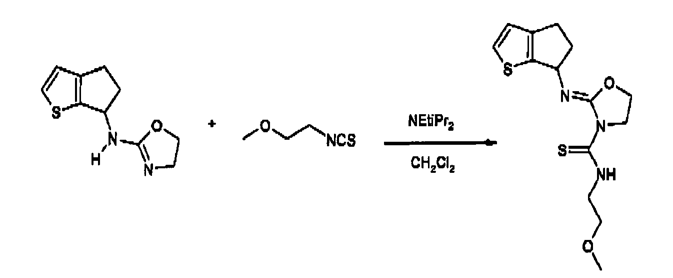

250 mg (1.2 mmol) N-(5,6-Dihydro-4H-cyclopenta[b]thien-6-yl)-4,5-dihydro-1,3-oxazol-2-amin sowie 144 mg (1.12mmol) N,N-Diisopropylethylamin werden in 25 ml Dichlormethan gelöst und 141mg (1.2 mmol) 2-Methoxyethylisothiocyanat in 2ml Dichlormethan zugetropft. Es wird 18 Stunden bei Raumtemperatur nachgerührt, die flüchtigen Bestandteile abdestilliert und über Kieselgel mit Cyclohexan/Essigester 5:1 chromatographiert, Es werden 150 mg (38% d.Th.) (2Z)-2-(5,6-Dihydro-4H-cyclopenta[b]thien-6-ylimino)-N-(2-methoxyethyl)-1,3-oxazolidine-3-carbothioamid isoliert.
Log P (sauer) 3,32 ¹H-NMR (DMSO-d6): 5.05 t

### Beispiel 4 (Vorstufe)

### 1-(2-Chloroethyl)-3-(6,7-dihydro-5H-cyclopenta[b]pyridin-5-yl)-harnstoff

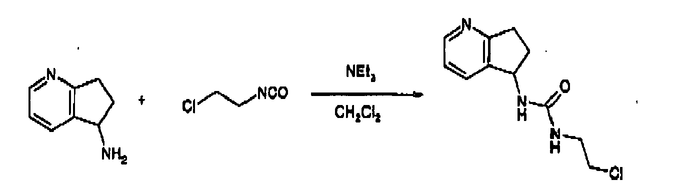

900 mg (6.7 mmol) 6,7-Dihydro-5H-cyclopenta[b]pyridin-5-amin und 814 mg (8.05 mmol) Triethylamin werden in 20 ml Dichlormethan gelöst auf 0°C gekühlt und 786 mg (7.45 mmol) 2-Chlorethylisocyanat in einem ml Dichlormethan zugetropft. Es wird 18 Stunden nachgerührt, die flüchtigen Bestandteile abdestilliert und der erhaltene Rückstand mit n-Hexan verrührt. Dabei fallen 1.5 g (93% d.Th.) 1-(2-Chloroethyl)-3-(6,7-dihydro-5H-cyclopenta[b]pyridin-5-yl)-hamstoff kristallin an.

Log P (sauer) 0.23 ¹H-NMR (CDCl₃); 3.6 m

### Beispiel 5

### N-(4,5-Dihydro-1,3-oxazol-2-yl)-6,7-dihydro-5H-cyclopenta[b]pyridin-5-amin

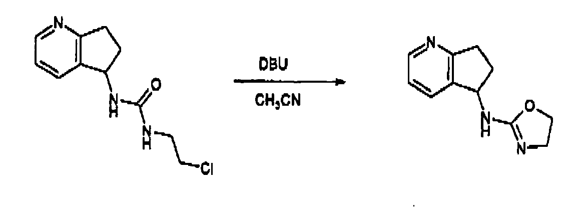

1.5 g (6.26 mmol) des 1-(6,7-Dihydro-5H-cyctopenta[b]pyridm-5-yl)-3'-(2-hydroxyethyl)-harnstoffs und 950 mg DBU in 30 ml Acetonitril warden 18 Stunden auf 60°C erwärmt. Die flüchtigen Bestandteile werden abdestilliert, der Rückstand in Essigsäureethylester gelöst und dreimal mit Wasser gewaschen. Nach dem Trocknen über Natriumsulfat wird filtriert und und evaporiert. Dabei werden 300 mg (24% d.Th.) des N-(4,5-Dihydro-1,3-oxazol-2-yl)-6,7-dihydro-5H-cyclopanta[b]pyridin-5-amin erhalten.
¹H-NMR (CDCl₃): 5.11 t

### Beispiel 6

### (2Z)-2-(6,7-Dihydro-5H-cyclopenta[b]pyridin-5-ylimino)-N-(2-methoxyethyl)-1,3-oxazolidine-3-carbothioamid

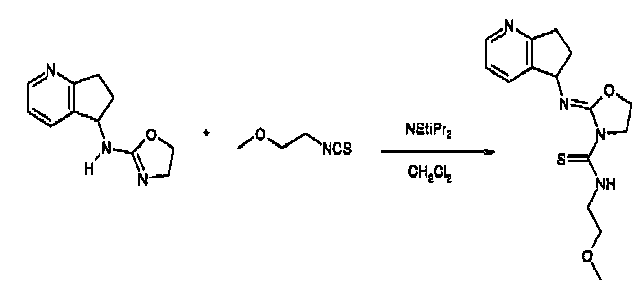

200 mg (0.98 mmol) N-(4,5-dihydro-1,3-oxazol-2-yl)-6,7-dihydro-5H-cyclopenta[b]pyridin-5-amin sowie 115 mg (0.98 mmol) 2-Methoxyethylisothiocyanat werden in 20 ml Dichlormethan vorgelegt und 118 mg (0.92 mmol) N,N-Diisopropylethylamin, gelöst in einem ml Dichlormethan, zugefügt. Es wird 18 Stunden bei Raumtemperatur nachgerührt und über Kieselgel (Cyclohexan/ Essigester 1/1) chromatographiert und damit 15 mg (5 % d.Th.) (2Z)-2-(6,7-Dihydro-5H-cyclopenta[b]pyridin-5-ylimino)-N-(2-methoxyethyl)-1,3-oxazolidine-3-carbothioamid isoliert.
Log P (sauer) 1.03 ¹H-NMR (CDCl₃): 5.11 t

**Tabelle 1**

| | | | |
|---|---|---|---|
| Verbindungen der Formel IA | | | |
| 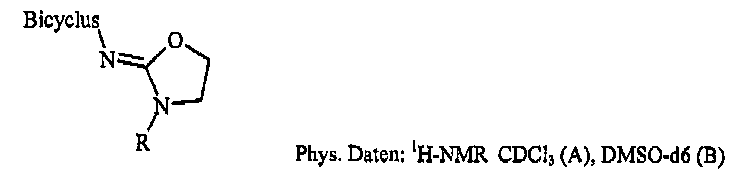 | | | |

| **Bsp.-Nr.** | **Bicyclus*** | **R**** | **Phys. Daten** |
|---|---|---|---|
| 2 | 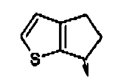 | H | 4.97 t (B) |
| 3 | 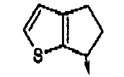 | C(S)-NH-CH₂-CH₂-OMe | 5.05 t (B) |
| 7 | 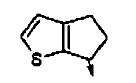 | C(S)-NH-CH₃ | 5.07 t (B) |
| 8 | 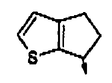 | C(S)-NH-CH₂-CF₃ | |
| 9 | 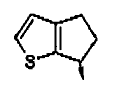 | C(S)-NH-CH₂-CH₃ | |
| 10 | 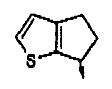 | C(S)-NH-iPr | |
| 11 | 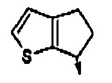 | P(S)(OMe)₂ | |
| 12 | 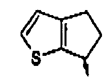 | C(O)CH₃ | |
| 13 | 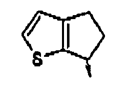 | C(O)OCH₃ | |
| 14 | 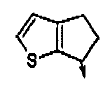 | C(S)-NH-CH₂-COOEt | 5.08 t (B) |
| 15 | 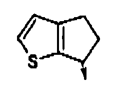 | C(S)-NH-CH₂-Ph | |
| 16 | 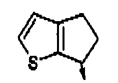 | C(S)-NH-CH₂-CH=CH₂ | |
| 17 | 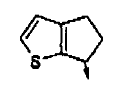 | C(S)-NH-CH₂-CH₂-SMe | |
| 18 | 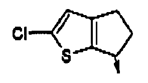 | H | 4.88 t (B) |
| 19 | 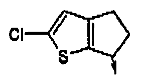 | C(S)-NH-CH₃ | 5.04 t (B) |
| 20 | 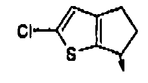 | C(S)-NH-CH₂-CH₂-OMe | 5.05 t (A) |
| 21 | 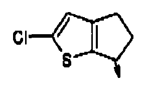 | C(S)-NH-CH₂-C≡CH | |
| 22 | 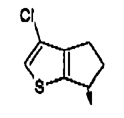 | H | |
| 23 | 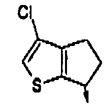 | C(S)-NH-CH₃ | |
| 24 | 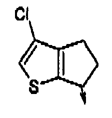 | C(S)-NH-CH₂-CH₂-OMe | |
| 25 | 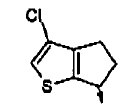 | 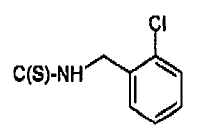 | |
| 26 | 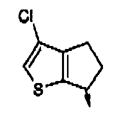 | 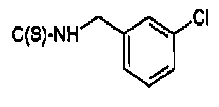 | |
| 27 | 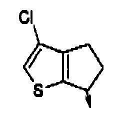 | 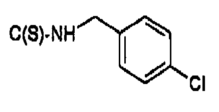 | |
| 28 | 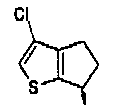 | 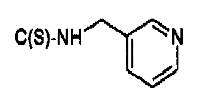 | |
| 29 | 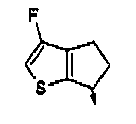 | H | |
| 5 | 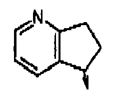 | H | 5.11 t (A) |
| 30 | 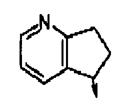 | C(S)-NH-CH₃ | 5.14 t (A) |
| 31 | 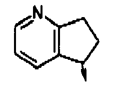 | C(S)-NH-CH₂-CF₃ | |
| 32 | 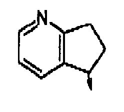 | C(S)-NH-CH₂-CH₃ | |
| 33 | 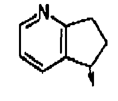 | C(S)-NH-iPr | |
| 34 | 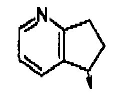 | P(S)(OMe)₂ | |
| 35 | 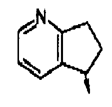 | C(O)CH₃ | |
| 36 | 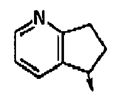 | C(O)OCH₃ | |
| 6 | 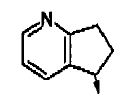 | C(S)-NH-CH₂-CH₂-OMe | 5.11 t (A) |
| 37 | 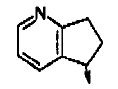 | C(S)-NH-CH₂-COOEt | |
| 38 | 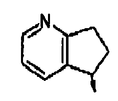 | C(S)-NH-CH₂-Ph | |
| 39 | 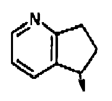 | C(S)-NH-CH₂-CH=CH₂ | |
| 40 | 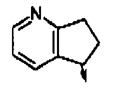 | C(S)-NH-CH₂-CH₂-SMe | |
| 41 | 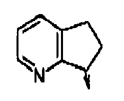 | H | |
| 42 | 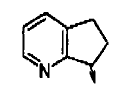 | C(S)-NH-CH₃ | |
| 43 | 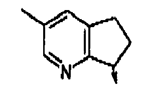 | H | 4.51 t (B) |
| 44 | 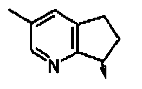 | C(S)-NH-CH₃ | 4.71 t (B) |
| 45 | 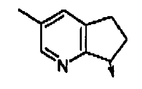 | C(S)-NH-CH₂-CH₂-OMe | 4.60 t (B) |
| 46 | 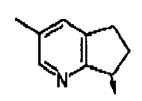 | C(S)-NH-CH₂-CF₃ | 4.73 t (B) |
| 47 | 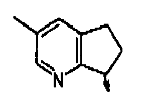 | C(S)-NH-CH₂-COOEt | 4.75 t (B) |
| 48 | 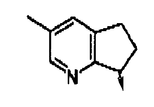 | C(S)-NH-CH₂-C≡CH | |
| 49 | 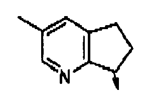 | H | |
| 50 | 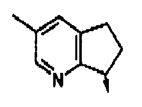 | C(S)-NH-CH₃ | |
| 51 | 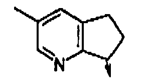 | C(S)-NH-CH₂-CH₂-OMe | |
| 52 | 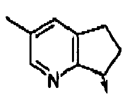 | 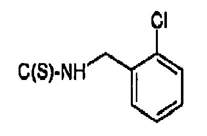 | |
| 53 | 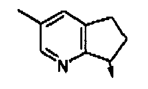 | 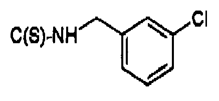 | |
| 54 | 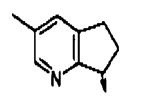 | 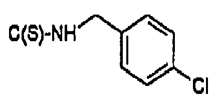 | |
| 55 | 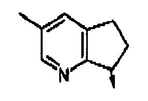 | 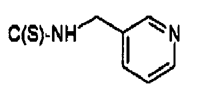 | |
| 56 | 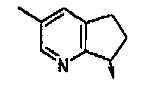 | H | |
| 57 | 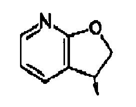 | H | 5,25-5,28 m (B) |
| 58 | 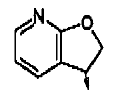 | C(S)-NH-CH₃ | |
| 59 | 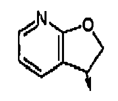 | C(S)-NH-CHa-CF₃ | |
| 60 | 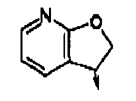 | C(S)-NH-CH₂-CH₃ | 1,07, t (B) |
| 61 | 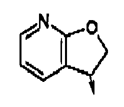 | C(S)-NH-iPr | |
| 62 | 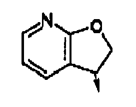 | P(S)(OMe)₂ | |
| 63 | 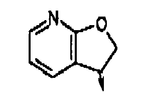 | C(O)CH₃ | |
| 64 | 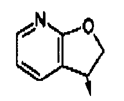 | C(O)OCH₃ | |
| 65 | 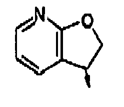 | C(S)-NH-CH₂-CH₂-Ome | 5,35-5,38 m (B) |
| 66 | 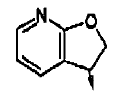 | C(S)-NH-CH₂-COOEt | |
| 67 | 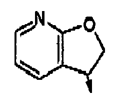 | C(s)-NH-CH₂-Ph | |
| 68 | 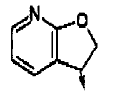 | C(S)-NH-CH₂-CH=CH₂ | |
| 69 | 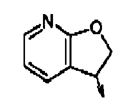 | C(S)-NH-CH₂-CH₂-SMe | |
| 70 | 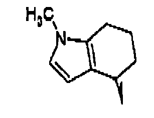 | H | |
| 71 | 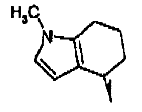 | C(S)-NH-CH₃ | |
| 72 | 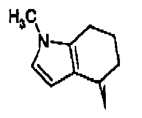 | C(S)-NH-CH₂-CF₃ | |
| 73 | 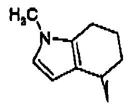 | C(S)-NH-CH₂-CH₃ | |
| 74 | 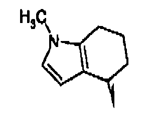 | C(S)-NH-iPr | |
| 75 | 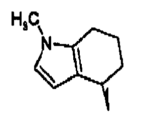 | P(S)(OMe)₂ | |
| 76 | 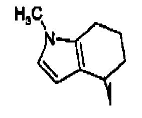 | C(O)CH₃ | |
| 77 | 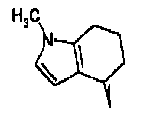 | C(O)OCH₃ | |
| 78 | 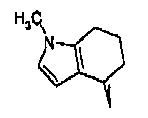 | C(S)-NH-CH₂-CH₂-OMe | |
| 79 | 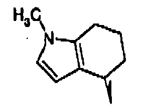 | C(S)-NH-CH₂-COOEt | |
| 80 | 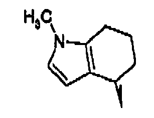 | C(S)-NH-CH₂-Ph | |
| 81 | 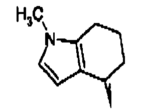 | C(S)-NH-CH₂-CH=CH₂ | |
| 82 | 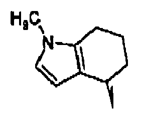 | C(S)-NH-CH₂-CH₂-SMe | |
| 83 | 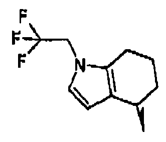 | H | |
| 84 | 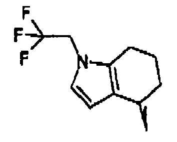 | C(S)-NH-CH₃ | |
| 85 | 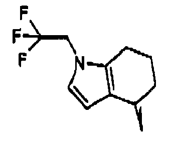 | C(S)-NH-CH₂-CF₃ | |
| 86 | 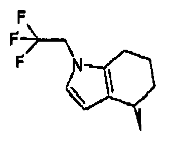 | C(S)-NH-CH₂-CH₃ | |
| 87 | 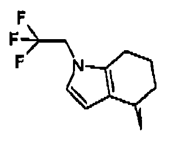 | C(S)-NH-iPr | |
| 88 | 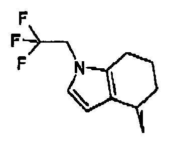 | P(S)(OMe)₂ | |
| 89 | 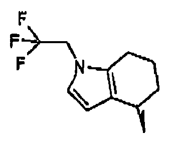 | C(O)CH₃ | |
| 90 | 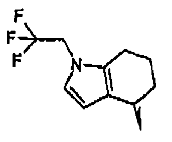 | C(O)OCH₃ | |
| 91 | 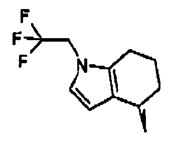 | C(S)-NH-CH₂-CH₂-OMe | |
| 92 | 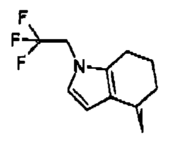 | C(S)-NH-CH₂-COOEt | |
| 93 | 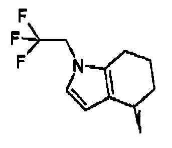 | C(S)-NH-CH₂-Ph | |
| 94 | 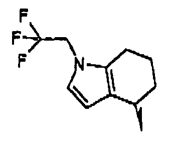 | C(S)-NH-CH₂-CH=CH₂ | |
| 95 | 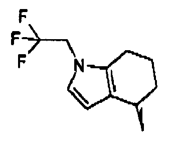 | C(S)-NH-CH₂-CH₂-SMe | |

| | | | |
|---|---|---|---|
| * der Pfeil kennzeichnet die Stelle der Verknüpfung des Bicyclus mit dem N-Atom. ** Me = Methyl Et = Ethyl iPr = iso-Propyl Ph = Phenyl | | | |

### Biologische Beispiele

### Beispiel Nr.1

### Myzus-Test (Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse, abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp Nr. 2, 3, 6, 7, 14, 18, 19, 30.

### Beispiel Nr. 2

### Myzus-Test - oral

Lösungsmittel: 80,0 Gewichtsteile Aceton

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit allen Stadien der Grünen Pfirsichblattlaus *(Myzus persicae)* besetzt, durch saugen an der Wirkstoffzubereitung der gewünschten Konzentration wird behandelt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr, 43, 44, 47.

## Patentansprüche

1. Verbindungen der Formel (I) 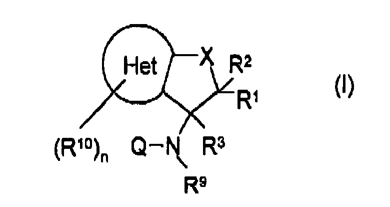 in welcher
Het für einen 5- oder 6-gliedrigen heterocyclischen Ring steht,
X für -O-, -SOₘ-, -NR⁴-, -CR⁵R⁶-, -CR⁵R⁶-CR⁷R⁸-, -O-CR⁷R⁸-, -SOₘ-CR⁷R⁸-, -NR⁴-CR⁷R⁸-, -CR⁵R⁶-O-, -CR⁵R⁶-SOₘ-, -CR⁵R⁶-NR⁴-, -SOₘNR⁴-, -NR⁴-SOₘ-, -NR⁴-O-oder -O-NR⁴- steht,
Q für eine der Gruppen A, B, C oder D steht 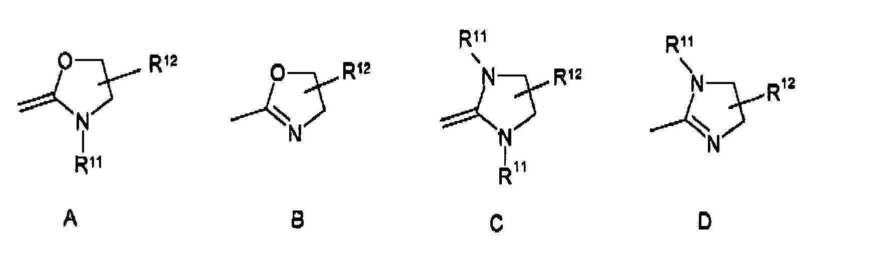
wobei
R⁹ in den Gruppen A und C mit dem verknüpfenden Kohlenstoffatom eine Doppel-bindung bildet,
oder
R⁹ für Wasserstoff, Cyano, Nitro, Hydroxy, Alkoxy, Amino, Alkylamino, Arylalkyl, Hatarylalkyl, Hetaryl, tri(C₁-C₆-Alkyl)silyl, -C(Y)R¹³, -C(Y)OR¹⁴, -C(Y)SR¹⁴,-SOₘR¹⁴, -C(Y)NR¹⁵R¹⁶, -SOₘNR¹⁵R¹⁶, -P(Y)(YR¹⁷)(OR¹⁸), P(Y)(NR¹⁹R²⁰)(NR²¹R²²), -CHR²³-Y-R²⁴, -CH=NR²⁵ oder -C(=NR²⁶)(SR²⁷) steht,
R¹, R², R⁴, R⁵, R⁶, R⁷ und R⁸ unabhängig von einander für Wasserstoff, Cyano, Nitro, Hydroxy oder für jeweils gegebenenfalls durch Halogen, Alkoxy oder Alkylthio substituiertes Alkyl, Alkenyl, Alkinyl, Alkoxy oder Alkylthio stehen,
R³ für Wasserstoff, Cyano, Carboxy, Carboxyalkyl, Carbamoyl, Carbamoylalkyl oder für jeweils gegebenenfalls durch Halogen, Alkoxy oder Alkylthio substituiertes Alkyl, Alkenyl oder Alkinyl steht,
R¹¹ für Wasserstoff, Cyano, Nitro, Hydroxy, Alkoxy, Amino, Alkylamino, Arylalkyl, Hetarylalkyl, Hetaryl, tri(C₁-C₆-Alkyl)silyl, -C(Y)R¹³, -C(Y)OR¹⁴, -C(Y)SR¹⁴,-SOₘR¹⁴, -C(Y)NR¹⁵R¹⁶, -SOₘNR¹⁵R¹⁶, -P(Y)(YR¹⁷)(OR¹⁸), P(Y)(NR¹⁹R²⁰)(NR²¹R²²), -CHR²³-Y-R²⁴, -CH=NR²⁵ oder -C(=NR²⁶)(SR²⁷) steht,
Y für Sauerstoff oder Schwefel steht,
R¹⁰ für Wasserstoff, Halogen, Cyano, Sauerstoff, Hydroxy, Alkyl, Alkoxy, Haloalkyl, Alkoxyalkyl, Alkoxyalkoxyallryl, Cycloalkyl, Cyanoalkyl, Haloalkoxy, Alkylthio, Haloalkylthio, Alkylsulfonyl, Alkylsulfonyloxy, Halogenalkylsulfonyl, Halogenalkylsufonyloxy, Alkoxycarbonyl, Acetyl, Alkylcarbonyl, Alkenylcarbonyl, Pentafluorosulfanyl, Amino, Mono- und Dialkylamino, Cycloalkylamino, Alkenyl, Haloalkenyl, Alkinyl, Haloalkinyl, Cyano oder Nitro oder für jeweils gegebenenfalls durch Halogen, Alkyl, Haloalkyl, Alkoxy, Haloalkoxy, Alkoxycarbonyl, Nitro oder Cyano substituiertes Aryl, Aryloxy oder Hetaryl steht,
n für eine ganze Zahl aus der Reihe 1, 2, 3 und 4 steht,
R¹² für Wasserstoff, Cyano, Halogen oder für gegebenenfalls durch Halogen, Alkoxy oder Alkylthio substituiertes Alkyl steht,
R¹³ und R¹⁴ unabhängig voneinander für Wasserstoff, Alkyl, Haloalkyl, Alkoxyalkyl, Alkoxyalkylcarbonyl, Alkenyl oder Alkinyl stehen,
R¹⁵ und R¹⁶ unabhängig voneinander für Wasserstoff oder für jeweils gegebenenfalls durch Halogen, Alkyl, Alkoxy, Cyano, Nitro, S(O)ₘ-alkyl, S(O)ₘ-Halogenalkyl substituiertes Alkyl, Cycloalkylalkyl, Alkylcycloalkyl, Alkenyl, Alkinyl, Arylalkyl, Hetcyclylalkyl, Heterearylalkyl, Aryl, Heterocyclyl, Hetaryl, Alkylcarbonyl, Alkoxycarbonyl, Alkoxycarbonylalkyl, Alkylthioalkyl, Alkylsulfinylalkyl, Alkylsulfonylalkyl, Mono- oder Dialkylaminocarbonylalkyl, Aminoalkyl, Mono oder Dialkylaminoalkyl oder Arylcarbonyl stehen,
R¹⁷ und R¹⁸ unabhängig voneinander für Wasserstoff, Alkyl, Haloalkyl, Alkenyl oder Alkinyl stehen,
R¹⁹, R²⁰, R²¹ und R²² unabhängig voneinander Wasserstoff, Alkyl oder Alkenyl stehen,
R²³ für Wasserstoff oder Alkyl steht,
R²⁴ für Alkyl, Haloalkyl, C(Y)R²⁸ oder P(Y)(OR²⁹)(OR³⁰) steht,
R²⁵ für Wasserstoff, Alkyl, Haloalkyl, Hydroxy, Alkoxy, Haloalkoxy, Cyano Hetarylalkyl oder Hetaryl steht,
R²⁶ und R²⁷ unabhängig voneinander für Wasserstoff, Alkyl, Aralkyl, -C(Y)R¹³,-C(Y)OR¹⁴, -C(Y)SR¹⁴, -SOₘR¹⁴, -C(Y)NR¹⁵R¹⁶, -SOₘNR¹⁵R¹⁶, -P(Y)(YR¹⁷)(OR¹⁸), P(Y)(NR¹⁹R²⁰)(NR²¹R²²) oder -CHR²³-Y-R²⁴ stehen,
R²⁸ für Wasserstoff, Alkyl oder Haloalkyl steht,
R²⁹ und R³⁰ unabhängig voneinander für Wasserstoff, Alkyl oder Haloalkyl stehen und
m für eine ganze Zahl aus der Reihe 0, 1 und 2 steht,
mit der Maßgabe, dass Het nicht für Furan oder Thiophen steht, wenn X für CR⁵R⁶CR⁷R⁸ und Q für die Gruppe B steht und R¹, R², R³, R⁵, R⁶, R⁷, R⁸, R⁹, R¹⁰ und R¹² gleichzeitig für Wasserstoff stehen.

2. Mittel, **gekennzeichnet durch** einen Gehalt von mindestens einer Verbindung der Formel (I) gemäß Anspruch 1 und üblichen Streckmitteln und/oder oberflächenaktiven Substanzen.

3. Verfahren zum Bekämpfen von Schädlingen **dadurch gekennzeichnet, dass** man eine Verbindung der Formel (I) gemäß Anspruch 1 oder ein Mittel gemäß Anspruch 6 auf die Schädlinge und/oder ihren Lebensraum einwirken lässt.

4. Verwendung von Verbindungen der Formel (I) gemäß Anspruch 1 oder von Mitteln gemäß Anspruch (1)2 zum Bekämpfen von Schädlingen.
